# EUROPEAN PATENT SPECIFICATION

(11) **EP 0 456 699 B1**
(45) Date of publication and mention of the grant of the patent: **26.04.1995**
(21) Application number: 90902641.1
(22) Date of filing: 09.02.1990
(51) Int. Cl.: G01N 33/80, G01N 33/48, G01N 33/53, B01L 3/00

(54) **TESTING OF LIQUIDS**
PRÜFUNG VON FLÜSSIGKEITEN
CONTROLE DE LIQUIDES

(30) Priority: 10.02.1989 GB 8903046
(43) Date of publication of application: 21.11.1991
(73) Proprietor: VALE, David Roger, Burton-on-Trent, Staffordshire DE15 0RD (GB)
(72) Inventor: VALE, David Roger, Burton-on-Trent, Staffordshire DE15 0RD (GB)
(74) Representative: Campbell, Iain Angus
(86) International application number: GB9000202
(87) International publication number: WO9009596

(56) References cited:
- EP-A- 79 861
- EP-A- 104 881
- EP-A- 138 377
- EP-A- 321 736
- EP-A- 340 562
- GB-A- 2 197 721

## Description

This invention relates to apparatus for, and a method of testing liquids, particularly but not exclusively blood.

Many immunological tests are performed by determining antibody/antigen reaction by the presence of agglutination. The physical method of agglutination involves overcoming the zeta potential of particles (the natural repelling force) to make them adhere to each other and so form a mass of particles. To enable these particles to adhere to each other and overcome their natural repelling force it is necessary to either reduce their zeta potential or to physically bridge the gap between the particles. If this is achieved then agglutination occurs with the consequence that large agglutinates become visible to the naked eye. A widely known example of this is in determining blood groups where agglutination of red cells (haemagglutination) determines the presence of an antigen or antibody.

A red blood cell that carries the 'A' antigen will cause haemagglutination in the presence of 'A' antibody (Anti-A). This antibody which generally has five binding sites will cause haemagglutination by one site binding onto a red cell with the 'A' antigen and another site binding with a similar adjacent red cell, the end result being bridges formed between masses of red cells to form an agglutinate clearly visible to the naked eye. The haemagglutination method has been and still is the only method used for determining blood groups. A certain combination of tests with Anti-A, Anti-B, Anti-AB and Anti-D will determine whether a blood sample is O, Rh positive etc. Blood grouping is not, of course, restricted to A,B,O and Rhesus, these being the most common determinations but can extend through the whole blood group system.

Most blood grouping requires a quite complex series of tests which already have been automated in many different forms - all of which detect the presence or absence of haemagglutination. For many years (at least 20) there has been a quick blood grouping system (Eldon cards) that have been used for confirmation grouping in the medical field as well as in education. Its principle is simple. Dried reagents are located on a card and a drop of blood is added to each reagent, this mixing causing the dried reagent to rehydrate and react with the red cells and agglutinate where appropriate; the pattern of agglutination determining the blood group. There is an increasing reluctance to use such an open system with the greater awareness of blood transmissable diseases.

Haemagglutination is not only resticted to blood groups but may be used in many serological tests. Hepatitis, syphilis and HIV (AIDS) tests may be performed using this method to determine the presence of the antibody to these diseases.

GB 2197721 discloses a slide for use in agglutinographic tests. This slide comprises a chamber defined between two plates with an inlet. Part of the chamber comprises a viewing area. A non-linear capillary channel is provided in the chamber extending from the inlet to the viewing area. Immunochemical particle reagents can be introduced into the chamber through the inlet such that if agglutination occurs this can be seen in the viewing area.

EP 0104881 discloses apparatus usable at a patient's bedside for blood testing. The apparatus comprises a well in which a porous plastic member can be dipped. Capillaries extend through the plastic member to an observation area such that if agglutination occurs the agglutinate will not reach the observation area. By using dyes in the antisera a colour signal is provided in the observation area indicating whether or not agglutination has taken place.

According to the present invention there is provided apparatus for testing for the presence of a substance in a liquid, the apparatus comprising means for locating a component which agglutinates with the substance, means for supplying the liquid to the locating means to mix with the component, passage means, which includes capillary means, reaching from said locating means and including a restriction to flow whereby agglutinated material causes a reduction in flow of liquid through the passage means characterised in that the passage means connects with an indicator chamber, the chamber being constructed such that presence of the mixture therein either makes a previously substantially invisible symbol visible or makes a previously visible symbol substantially invisible whereby in the absence of agglutination the mixture reaches the indicator chamber changing the indication provided thereby.

The passage means may include a capillary tube along the whole length of which the mixture travels only in the absence of the agglutination.

The diameter of the capillary tube preferably decreases as it extends away from the locating means.

Desirably the capillary tube extends directly from the locating means to urge the liquid through the locating means by capillary action.

Alternatively or in addition the passage means includes a porous member with a pore size such that the mixture may only pass therethrough when substantially no agglutination occurs, and hence none of the substance is present.

The apparatus preferably comprises a plurality of locating means, passage means and indicator chambers, each passage means connecting with a different indicator chamber such that the presence of a plurality of substances can be simultaneously tested, each locating means including a different agglutinating component.

In such an apparatus each symbol is preferably different, and desirably one of the locating means and respective passage means is a control such that no component, or a component which does not agglutinate with any substance in the liquid or the liquid itself, is used in the locating means.

The apparatus preferably comprises a collecting chamber connected to the or each locating means into which the liquid can be introduced. The collecting chamber and the or each locating means are preferably connected by a capillary tube such that the liquid is urged by capillary action in to the or each locating means.

If the apparatus is to be used for testing blood, the components are preferably monoclonal antibodies, to which an anti-coagulant may have been added.

The apparatus is preferably integrally formed and may be made of a transparent plastics material.

Also according to the present invention there is provided a method for testing for the presence of a substance in a liquid, the method comprising adding the liquid to a component which either agglutinates with the substance, passing the mixture through a restricted passage means by capillary action whereby the flow of liquid is controlled in accordance with the degree of agglutination, characterised in that if agglutination does not occur upon adding the liquid to a component either a previously substantially invisible symbol is made visible or a previously visible symbol is made substantially invisible.

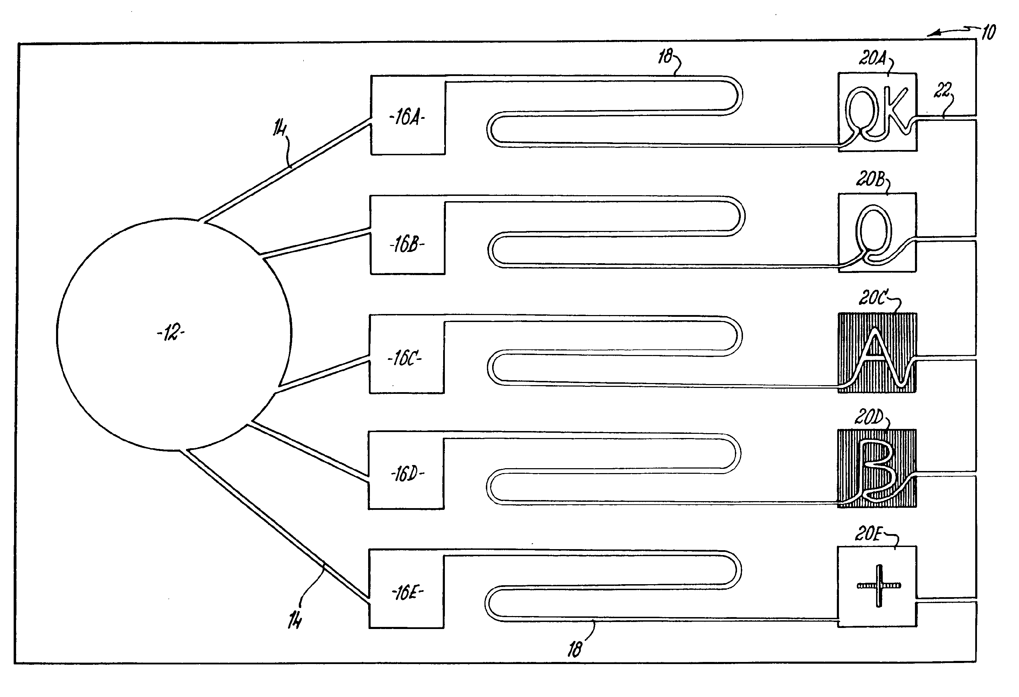

An embodiment of the present invention will now be described by way of example only with reference to the single figure of the accompanying drawings which shows a plan view of a blood testing apparatus.

The drawing shows an apparatus 10 suitable for testing blood to give an indication of blood type. The apparatus 10 comprises a single substantially rectangular transparent component, made, for example, by moulding a plastics material. The component 10 has a hollow 12 formed in the upper surface in use thereof, towards one of its ends. The hollow 12 is of a size to accept a drop of liquid. Within the component 10 leading from the hollow 12 towards the other end, are five capillaries 14, each 14 leading to a respective one of five chambers or locating means 16A, 16B, 16C, 16D, 16E.

Each chamber 16 contains a permeable membrane (not shown) which is impregnated with anti-coagulant. The membranes in chambers 16B, 16C, 16D, 16E are also impregnated respectively with monoclonal antibodies, namely Anti-AB, Anti-A, Anti-B and Anti-D.

Further capillaries 18 lead from the downstream side of each of the chambers 16, to respective indication chambers 20A,B,C,D,E. The diameters of the capillaries 18 reduce as they extend from the chambers 16 and have the shape of a reverse squashed 'S' . Vent capillaries 22 lead from the opposite side of the chambers 20 and connect with atmosphere. The chambers 20 are constructed to either display an otherwise substantially invisible symbol, or to make an otherwise visible symbol substantially invisible, upon blood entering therein. The chamber 20A comprises a capillary arrangement shaped to form the symbol 'OK' . The chamber 20B includes an arrangement to provide the symbol 'O' . Chambers 20C and 20D have capillary arrangements to respectively show the symbols 'A' and 'B' and these are provided against a red background such that they are visible only if the capillary is empty. Chamber 20E has a red cross marked on it which is visible unless the chamber E is full of blood.

In use, a drop of blood is placed in the hollow 12. Capillary action causes blood from the hollow 12 to pass up the capillaries 14 into the chambers 16 to mix with the reagents impregnated in the membranes. The mixtures thus formed are urged by capillary action along the capillaries 18. The converging of the capillaries 18 causes further mixing of the mixtures. If agglutination occurs in the mixture this will tend to block the respective capillary 18 and thus the flow of blood will not reach the respective chamber 20. If blood reaches the respective chamber 20 it either causes the message already thereon to be obscured, or causes a message to be visible thereon.

The chambers 16A and 20A and respective capillaries 14,18,22, act as a control, and blood should always flow into the chamber 20A making the symbol 'OK' visible and thus indicating that the apparatus 10 is functioning correctly. The chambers 16B and 20B and respective capillaries 14,18,22 indicate whether or not the blood group is O, i.e. if agglutination does not occur with Anti-AB then blood will flow in the capillary 18 leading to chamber 20B to highlight the 'O' symbol. The chambers 16C and 20C, and 16D and 20D, act in a similar manner respectively for blood groups A and B, but in these instances the A or B symbol is highlighted if agglutination does occur. Chambers 16E and 20E show whether the blood is positive and this symbol will remain visible if agglutination with Anti-D occurs.

There is thus described a relatively simple system for automatically providing a readily visible indication of blood type. The apparatus of the invention can be inexpensively manufactured due to its simplicity of construction. The system does not require significant handling of the blood being tested with the risks involved therewith.

Various modifications may be made without departing from the scope of the invention as defined by the claims. For example it may not be necessary for the capillaries 18 to narrow. A membrane or other filter device which would block mixtures in which agglutination has occured and allow free passage for other mixtures, could be used instead of or within the capillaries 18. Different methods could be used to illustrate whether agglutination has taken place.

A method of 'washing' a material, e.g. red blood cells, may be provided in which a reagent is passed through a permeable membrane which does not allow passage of the material, through the material into a reservoir. In the case of red blood cells saline solution may be passed through to remove serum previously added to the cells to allow passage of immunoglobulins as would be used in a Coombs test (Antiglobulin test).

Apparatus such as described can be used for many other medical tests. Obviously for such tests different relevant reagents will be provided in the chambers, and a different number of chambers may be required. This type of apparatus could be used for testing in the following fields.
Rheumatoid Arthritis
Pregnancy testing
Glandular Fever
Bacterial Identification
S.L.E
Hepatitis
H.I.V.
C.M.V.
Thyroid Antibodies
F.D.P.
Antibody screening
Direct antiglobulin testing

It would be possible to have more than one chamber containing reagents, whereby more than one reagent could be sequentially added. A network of branching could be used to facilitate various mixing alternatives. Further, a collecting chamber could be provided at the end of the system.

## Claims

1. Apparatus for testing for the presence of a substance in a liquid, the apparatus comprising means (16) for locating a component which agglutinates with the substance, means (12,14) for supplying the liquid to the locating means (16) to mix with the component, passage means (18), which includes capillary means, reaching from said locating means (16) and including a restriction to flow whereby agglutinated material causes a reduction in flow of liquid through the passage means (18) characterised in that the passage means (18) connects with an indicator chamber (20), the chamber (20) being constructed such that presence of the mixture therein either makes a previously substantially invisible symbol visible or makes a previously visible symbol substantially invisible whereby in the absence of agglutination the mixture reaches the indicator chamber (20) changing the indication provided thereby.

2. Apparatus according to claim 1, characterised in that the passage means (18) includes a capillary tube (18) along the whole length of which the mixture travels only in the absence of agglutination.

3. Apparatus according to claim 2, characterised in that the diameter of the capillary tube (18) decreases as it extends away from the locating means (16).

4. Apparatus according to claim 2 or claim 3, characterised in that the capillary tube (18) extends directly from the locating means (16) to urge the liquid through the locating means (16) by capillary action.

5. Apparatus according to any of the preceding claims, characterised in that the passage means (18) includes a porous member with a pore size such that the mixture may only pass therethrough when substantially no agglutination occurs, and hence none of the substance is present.

6. Apparatus according to any of the preceding claims, characterised in that the apparatus comprises a plurality of locating means (16), passage means (18), and indicator chambers (20), each passage means (18) connecting with a different indicator chamber (20) such that the presence of a plurality of substances can be simultaneously tested, each locating means (16) including a different agglutinating component.

7. Apparatus according to claim 6, characterised in that each symbol is different.

8. Apparatus according to any of claims 6 or 7, characterised in that one of the locating means (16) and respective passage means (18) is a control such that no component, or a component which does not agglutinate with any substance in the liquid or the liquid itself, is used in the locating means (16).

9. Apparatus according to any of the preceding claims, characterised in that the apparatus comprises a collecting chamber (12) connected to the or each locating means (16) into which the liquid can be introduced.

10. Apparatus according to claim 9, characterised in that the collecting chamber (12) and the or each locating means (16) are connected by a capillary tube (14) such that the liquid is urged by capillary action into the or each locating means (16).

11. Apparatus according to any of the preceding claims, characterised in that the component or at least one of the components is a monoclonal antibody.

12. Apparatus according to claim 11, characterised in that an anti-coagulant has been added to the or each component.

13. Apparatus according to any of the preceding claims, characterised in that the apparatus is integrally formed.

14. Apparatus according to any of the preceding claims, characterised in that the apparatus is made of a transparent plastics material.

15. A method for testing for the presence of a substance in a liquid, the method comprising adding the liquid to a component which agglutinates with the substance, passing the mixture through a restricted passage means (18) by capillary action whereby the flow of liquid is controlled in accordance with the degree of agglutination, characterised in that if agglutination does not occur upon adding the liquid to a component either a previously substantially invisible symbol is made visible or a previously visible symbol is made substantially invisible.

16. A method according to claim 15, characterised in that the passage means (18) includes a capillary tube (18) along the whole length of which the mixture travels only in the absence of the agglutination.

17. A method according to claim 15 or 16, characterised in that the liquid is simultaneously added to separate different components.

18. A method for testing blood, characterised in that separate portions of the blood are combined with different monoclonal antibodies and the mixtures formed are passed through a restricted passage (18) by capillary action such that if agglutination occurs flow ceases in the passage and if no agglutination occurs either a previously invisible symbol is made visible or a previously visible symbol is made substantially invisible thereby indicating the blood type.

## Patentansprüche

1. Vorrichtung zum Testen des Vorhandenseins einer Substanz in einer Flüssigkeit mit Mitteln (16) zur Aufnahme einer mit der Substanz agglutinierenden Komponenten, Mitteln (12, 14) zum Zuführen der Flüssigkeit zu den Aufnahmemitteln (16) zum Mischen mit der Komponenten, Kapillarmittel enthaltenden Durchgangsmitteln (18), die von den Aufnahmemitteln (16) herkommen und eine Durchflußverengung enthalten, wobei agglutiniertes Material eine Reduzierung des Durchflusses von Flüssigkeit durch die Durchgangs mittel (18) verursacht, dadurch gekennzeichnet, daß die Durchgangsmittel (18) mit einer Indikatorkammer (20) in Verbindung stehen, die derart konstruiert ist, daß das Vorhandensein der Mischung in ihr entweder ein zuvor im wesentlichen unsichtbares Symbol sichtbar macht oder ein zuvor sichtbares Symbol im wesentlichen unsichtbar macht, wobei bei Fehlen einer Agglutination die Mischung die Indikatorkammer (20) erreicht und dabei die Indikation ändert.

2. Vorrichtung nach Anspruch 1, dadurch gekennzeichnet, daß die Durchgangsmittel (18) ein Kapillarrohr (18) enthalten, wobei die Mischung nur beim Fehlen einer Agglutination entlang der gesamten Rohrlänge strömt.

3. Vorrichtung nach Anspruch 2, dadurch gekennzeichnet, daß der Durchmesser des Kapillarrohrs (18) mit der Entfernung von den Aufnahmemitteln abnimmt.

4. Vorrichtung nach Anspruch 2 oder Anspruch 3, dadurch gekennzeichnet, daß das Kapillarrohr (18) direkt von den Aufnahmemitteln (16) ausgeht, um die Flüssigkeit durch Kapillarwirkung durch die Aufnahmemittel (16) zu treiben.

5. Vorrichtung nach einem der vorstehenden Ansprüche, dadurch gekennzeichnet, daß die Durchgangsmittel (18) ein poröses Glied mit einer solchen Porengröße enthalten, daß die Mischung nur hindurchgelangen kann, wenn im wesentlichen keine Agglutination auftritt und somit nichts von der Substanz vorhanden ist.

6. Vorrichtung nach einem der vorstehenden Ansprüche, dadurch gekennzeichnet, daß sie eine Mehrzahl von Aufnahmemitteln (16), Durchgangsmitteln (18) und Indikatorkammern (20) enthält, wobei jedes Durchgangsmittel (18) mit einer verschiedenen Indikatorkammer (20) in Verbindung steht, so daß das Vorhandensein einer Mehrzahl von Substanzen gleichzeitig getestet werden kann, wobei jedes Aufnahmemittel (16) eine verschiedene Agglutinationskomponente enthält.

7. Vorrichtung nach Anspruch 6, dadurch gekennzeichnet, daß jedes Symbol verschieden ist.

8. Vorrichtung nach einem der Ansprüche 6 oder 7, dadurch gekennzeichnet, daß eines der Aufnahmemittel und jeweiliges Durchgangsmittel (18) eine Kontrolle darstellt, derart, daß in dem Aufnahmemittel (16) keine Komponente oder eine Komponente, die mit keiner Substanz in der Flüssigkeit oder der Flüssigkeit selbst agglutiniert, verwendet wird.

9. Vorrichtung nach einem der vorstehenden Ansprüche, dadurch gekennzeichnet, daß sie eine mit dem oder jedem Aufnahmemittel (16) verbundene Sammelkammer (12) enthält, in die die Flüssigkeit eingebracht werden kann.

10. Vorrichtung nach Anspruch 9, dadurch gekennzeichnet, daß die Sammelkammer (12) und das oder jedes Aufnahmemittel (16) durch ein Kapillarrohr (14) verbunden sind, so daß die Flüssigkeit durch Kapillarwirkung in das oder jedes Aufnahmemittel (16) getrieben wird.

11. Vorrichtung nach einem der vorstehenden Ansprüche, dadurch gekennzeichnet, daß die Komponente oder mindestens eine der Komponenten ein monoklonaler Antikörper ist.

12. Vorrichtung nach Anspruch 11, dadurch gekennzeichnet, daß ein Antikoagulans zu der oder jeder Komponenten hinzugefügt worden ist.

13. Vorrichtung nach einem der vorstehenden Ansprüche, dadurch gekennzeichnet, daß sie einstückig ausgebildet ist.

14. Vorrichtung nach einem der vorstehenden Ansprüche, dadurch gekennzeichnet, daß sie aus transparentem Kunststoffmaterial hergestellt ist.

15. Verfahren zum Testen des Vorhandenseins einer Substanz in einer Flüssigkeit, bei dem die Flüssigkeit einer mit der Substanz agglutinierenden Komponenten hinzugefügt und die Mischung durch Kapillarwirkung durch ein verengtes Durchgangsmittel (18) geleitet wird, wodurch der Flüssigkeitsdurchfluß in übereinstimmung mit dem Agglutinationsausmaß kontrolliert wird, dadurch gekennzeichnet, daß, wenn nach dem Hinzufügen der Flüssigkeit zu einer Komponenten keine Agglutination auftritt, entweder ein zuvor im wesentlichen unsichtbares Symbol sichtbar gemacht wird oder ein zuvor sichtbares Symbol im wesentlichen unsichtbar gemacht wird.

16. Verfahren nach Anspruch 15, dadurch gekennzeichnet, daß die Durchgangsmittel (18) ein Kapillarrohr (18) enthalten, wobei die Mischung nur beim Fehlen einer Agglutination entlang der gesamten Rohrlänge strömt.

17. Verfahren nach Anspruch 15 oder 16, dadurch gekennzeichnet, daß die Flüssigkeit gleichzeitig zu separaten verschiedenen Komponenten hinzugefügt wird.

18. Verfahren zum Testen von Blut, dadurch gekennzeichnet, daß separate Portionen von Blut mit verschiedenen monoklonalen Antikörpern kombiniert und die gebildeten Mischungen durch Kapillarwirkung durch einen verengten Durchgang (18) geleitet werden, so daß, wenn Agglutination auftritt, der Durchfluß in dem Durchgang aufhört und, wenn keine Agglutination auftritt, entweder ein zuvor unsichtbares Symbol sichtbar gemacht wird oder ein zuvor sichtbares Symbol unsichtbar gemacht wird, wodurch der Bluttyp angegeben wird.

## Revendications

1. Appareil servant à détecter la présence d'une substance dans un liquide, comprenant des moyens (16) de localisation d'un composant qui s'agglutine avec la substance, des moyens (12, 14) servant à alimenter les moyens (16) de localisation en liquide pour que celui-ci se mélange avec le composant, des passages (18), comprenant des éléments capillaires, s'étendant à partir des moyens (16) de localisation et comprenant un élément réducteur de flux, le matériau agglutiné entraînant une réduction du flux de liquide à travers les passages (18), caractérisé en ce que les passages (18) sont reliés à une chambre indicatrice (20), la chambre (20) étant conçue de telle sorte que la présence du mélange à l'intérieur de celle-ci fait soit apparaître un symbole sensiblement invisible auparavant, soit sensiblement disparaître un symbole visible auparavant, le mélange, en l'absence d'agglutination, arrivant dans la chambre indicatrice (20) en modifiant l'indication qu'elle donne.

2. Appareil selon la revendication 1, caractérisé en ce que les passages (18) comprennent un tube capillaire (18) sur toute la longueur duquel le mélange s'écoule uniquement en l'absence d'agglutination.

3. Appareil selon la revendication 2, caractérisé en ce que le diamètre du tube capillaire (18) va en diminuant plus son éloignement par rapport aux moyens (16) de localisation augmente.

4. Appareil selon la revendication 2 ou 3, caractérisé en ce que le tube capillaire (18) s'étend directement à partir des moyens (16) de localisation, afin que le liquide passe à travers les moyens (16) de localisation par capillarité.

5. Appareil selon l'une quelconque des revendications précédentes, caractérisé en ce que les passages (18) comprennent un élément poreux dont la taille des pores est telle que le mélange ne peut le traverser que lorsqu'il n'y a sensiblement pas d'agglutination et que, donc, il n'y a aucune substance.

6. Appareil selon l'une quelconque des revendications précédentes, caractérisé en ce que l'appareil comprend une pluralité de moyens (16) de localisation, des passages (18) et des chambres indicatrices (20), chaque passage (18) étant relié à une chambre indicatrice (20) différente de telle sorte que la présence d'une pluralité de substances puisse être testée simultanément, chaque moyen (16) de localisation comprenant un composant d'agglutination différent.

7. Appareil selon la revendication 6, caractérisé en ce que chaque symbole est différent.

8. Appareil selon l'une quelconque des revendications 6 ou 7, caractérisé en ce que l'un des moyens (16) de localisation et des passages (18) respectifs est constitué d'un organe de commande faisant en sorte qu'aucun composant, ou un composant ne s'agglutinant pas avec toute susbtance présente dans le liquide ou avec le liquide proprement dit, n'est utilisé dans les moyens (16) de localisation.

9. Appareil selon l'une quelconque des revendications précédentes, caractérisé en ce que l'appareil comprend une chambre collectrice (12) reliée au moyen ou à chaque moyen (16) de localisation dans lequel le liquide peut être introduit.

10. Appareil selon la revendication 9, caractérisé en ce que la chambre collectrice (12) et le ou chaque moyen (16) de localisation sont reliés par un tube capillaire (14) de telle sorte que le liquide circule par capillarité vers le ou chaque moyen (16) de localisation.

11. Appareil selon l'une quelconque des revendications précédentes, caractérisé en ce que le composant ou au moins l'un des composants est un anticorps monoclonal.

12. Appareil selon la revendication 11, caractérisé en ce qu'un anti-coagulant a été ajouté au composant ou à chaque composant.

13. Appareil selon l'une quelconque des revendications précédentes, caractérisé en ce que l'appareil ne forme qu'une seule pièce.

14. Appareil selon l'une quelconque des revendications précédentes, caractérisé en ce que l'appareil est constitué d'un matériau en plastique transparent.

15. Procédé pour détecter la présence d'une substance dans un liquide, le procédé comprenant le fait d'ajouter le liquide à un composant qui s'agglutine avec la substance, de faire circuler le mélange à travers un passage (18) rétréci, par capillarité, le débit de liquide étant commandé en fonction du taux d'agglutination, caractérisé en ce que, s'il n'y a pas d'agglutination lorsqu'on ajoute le liquide à un composant, soit un symbole sensiblement invisible auparavant apparaît, soit un symbole visible auparavant disparaît sensiblement.

16. Procédé selon la revendication 15, caractérisé en ce que les passages (18) comprennent un tube capillaire (18) sur toute la longueur duquel le mélange s'écoule uniquement en l'absence d'agglutination.

17. Procédé selon la revendication 15 ou 16, caractérisé en ce que le liquide est simultanément ajouté à différents composants séparés.

18. Procédé de test du sang, caractérisé en ce que des parties séparées du sang sont combinées avec différents anticorps monoclonaux et les mélanges constitués sont amenés à passer à travers un passage (18) rétréci, par capillarité, de telle sorte que, s'il y a agglutination, le flux s'interrompt dans le passage et que, s'il n'y a pas d'agglutination, soit un symbole sensiblement invisible auparavant apparaît, soit un symbole visible auparavant disparaît sensiblement, ce qui donne une indication sur le type de sang.
